# EUROPEAN PATENT APPLICATION

(11) **EP 3 824 870 A1**
(43) Date of publication of application: **26.05.2021**
(21) Application number: 20208608.8
(22) Date of filing: 19.11.2020
(51) Int. Cl.: A61K 8/02, A61K 8/34, A61K 8/39, A61K 8/49, A61K 8/55, A61K 8/92, A61Q 19/00, A41D 19/00, A61F 7/00

(54) **ELASTOMERIC ARTICLE CONTAINING A COATING COMPOSITION**

(30) Priority: 20.11.2019 MY PI2019006807
(71) Applicant: Top Glove International SDN. BHD., Klang, Selangor 41050 (MY)
(72) Inventor: WONG, Chong Ban, 41050 Klang, Selangor (MY); LING, Siew Szen, 41050 Klang, Selangor (MY)
(74) Representative: Schiweck Weinzierl Koch Patentanwälte Partnerschaft mbB

(57) **Abstract**

A coating composition, an elastomeric article having the coating composition and a process for making coated elastomeric articles are disclosed in the present invention. The coating composition comprises 0.5% to 11% by weight/volume of at least one cooling agent; 0.5% to 11% by weight/volume of at least one non-ionic surfactant; and water. Preferred cooling agents are essential peppermint oil, menthol crystals and menthol flakes. The coated elastomeric articles prepared according to the present invention improve user's sensory experience of wearing the elastomeric articles for long durations without varying the user's hand temperature or limiting mobility of the user's hand.

## Description

### FIELD OF THE INVENTION

The present invention relates to an elastomeric article and a coating composition applied thereon on a skin-contacting surface of the elastomeric article to provide a cooling sensation to the skin.

### BACKGROUND OF THE INVENTION

Gloves are extensively used in various applications, both commercial and domestic, to protect arms and/or hands of users from physical harm, exposure to harmful chemicals or extreme conditions. Gloves are also used for maintaining hygiene while handling edibles or while using for medical treatment purposes.

Some of the aforementioned applications require users to wear gloves or finger cots continuously for long durations, making the wearing of these elastomeric articles uncomfortable. The tight fit and the material of the elastomeric article lead to limited mobility of the user's hand and excess perspiration which causes an unpleasant sensory experience for the user. This can even lead to skin problems due to a moist environment created by perspiration.

There have been attempts in the prior art to make the wearing of elastomeric articles more comfortable by controlling perspiration without limiting grip and mobility of the user's hand. However, the prior art uses refrigerants in the composition for preparing the elastomeric articles such as gloves, which cause an actual fall in the temperature of the user's hand when it comes in contact with the glove. Hence, the overall experience may not be comfortable as the hand may be cold due to use of the refrigerants.

There is therefore a need for a solution to improve the user's sensory experience of wearing elastomeric articles without varying the hand temperature or limiting mobility of the user's hand and yet making the wearing experience comfortable.

### SUMMARY OF THE INVENTION

According to one aspect of the present invention, an elastomeric article having at least one skin-contacting surface coated with a water-based coating composition is disclosed, wherein the coating composition comprises 0.5% to 11% by weight/volume of at least one cooling agent; 0.5% to 11% by weight/volume of at least one non-ionic surfactant; and water, wherein preferably the rest of the coating composition (that has been formed from a coating solution) is made of water.

According to the present invention, the coating (formed by the coating composition) on the skin-contacting surface of the elastomeric article may have a thickness in the range of 0.1 microns to 1.2 microns.

Further, the cooling agent and the non-ionic surfactant may be present in the composition in a ratio of about 1:1.

Still further, the cooling agent may be selected from essential peppermint oil, menthol crystals, or menthol flakes or mixtures thereof. The non-ionic surfactant may be selected from polysorbate 20, octylphenoxypoly (ethyleneoxy) ethanol, branched and/or liquid lecithin or mixtures of any of these non-ionic surfactants.

According to a second aspect of the present invention, a process for making coated elastomeric articles using a coating composition is disclosed. The process may comprise the steps of former cleaning, coagulant dipping, drying, first latex dipping, drying, second latex dipping, drying, pre-leaching, beading, drying, cooling, chlorinating, post-leaching, coating with the coating composition, drying and stripping the elastomeric article from the former, wherein the coating is carried out online.

Further, the step of coating may include spraying a solution containing the coating composition on a skin-contacting surface of the elastomeric article, wherein temperature of the solution is maintained in the range between 20°C to 55°C and the spraying is carried out for a period between 1 second to 5 seconds.

Alternatively, the step of coating may include dipping a skin-contacting surface of the elastomeric article into a solution containing the coating composition, wherein temperature of the solution is maintained in the range between 25°C to 65°C and the dipping is carried out for a period between 5 seconds to 10 seconds.

Additional aspects, features and advantages of the invention will become apparent to those skilled in the art upon consideration of the following detailed description of preferred embodiments of the invention in conjunction with the drawing listed below.

### DETAILED DESCRIPTION OF THE INVENTION

Detailed description of preferred embodiments of the present invention is disclosed herein. It should be understood, however, that the embodiments are merely exemplary of the present invention, which may be embodied in various forms. Therefore, the details disclosed herein are not to be interpreted as limiting, but merely as the basis for the claims and for teaching one skilled in the art of the invention. The numerical data or ranges used in the specification are not to be construed as limiting.

The present invention discloses a coating composition for elastomeric articles and a process for making coated elastomeric articles wherein a coating formed from a coating composition is applied to a skin-contacting surface of the elastomeric article. Particularly, the present invention discloses a coating composition for elastomeric gloves and finger cots, wherein the elastomer is selected from natural rubber, nitrile rubber, polychloroprene, butyl rubber, thermoplastic elastomer (TPE), polyisoprene or combinations thereof.

According to the present invention, the coating composition provides a cooling sensation as essential peppermint oil is incorporated in the elastomeric gloves and finger cots, wherein the essential peppermint oil is extracted from a peppermint plant for use in the coating composition. Essential peppermint oil includes a naturally occurring organic compound in peppermint called menthol. Menthol binds to a cold-sensitive receptor protein (TRPM8) in the skin and produces the "cooling" effect without any actual fall in the hand temperature, thereby making the experience of wearing of the elastomeric article comfortable without limiting the hand functionality or mobility of the user's hand.

Embodiments of the present invention are directed towards a coating composition, an elastomeric article having the coating composition and a process for making coated elastomeric articles wherein a coating is formed when the coating composition (that is formed from a (coating) solution) is applied to a skin-contacting surface of the elastomeric article.

Hereinafter working of the present invention will be explained with respect to a coating composition used for coating gloves and finger cots; however, teachings of the present invention can be adapted for any other elastomeric articles which exhibit similar elastomeric characteristics such as dental dams, condoms, exercise bands and the like.

According to the present invention, the process for making coated elastomeric gloves and finger cots includes the steps of former cleaning, coagulant dipping, drying, first latex dipping, drying, second latex dipping, drying, pre-leaching, beading, drying, cooling, chlorinating, post-leaching, coating with the coating composition, drying and stripping the elastomeric article from the former.

The coating as proposed by the present invention is carried out online, wherein the coating is either sprayed on the elastomeric article or the elastomeric article is dipped into a solution containing the coating composition on the donning side or the skin-contacting surface of the elastomeric article on a former after a post-leaching process. As seen in TABLE 1 below, if the solution containing the coating composition is sprayed onto the skin-contacting surface of the elastomeric article, then the temperature of the solution is maintained in the range between 20°C to 55°C and the spraying is carried out for a period between 1 second to 5 seconds. Preferably, the temperature of the solution is maintained in the range between 25°C to 50°C and the spraying is carried out for a period between 2 seconds to 4 seconds. Typically, the temperature of the solution is maintained at 40°C and the spraying is carried out for 3 seconds.

Alternatively, as seen in TABLE 1, if the skin-contacting surface of the elastomeric article is dipped into a solution containing the coating composition, then the temperature of the solution is maintained in the range between 25°C to 65°C and the dipping is carried out for a period between 5 seconds to 10 seconds. Preferably, the temperature of the solution is maintained in the range between 30°C to 55°C and the dipping is carried out for a period between 6 seconds to 8 seconds. Typically, the temperature of the solution is maintained at 45°C and the dipping is carried out for 7 seconds as seen in TABLE 1.

**TABLE 1: Process parameters involved in applying a coating to a skin-contacting surface of a glove**

| **Process type** | **Preferred range** | **Working range** | **Typical value** |
|---|---|---|---|
| Spraying using a solution containing the coating composition | | | |
| a) Temperature (°C) | a) 25 to 50 | a) 20 to 55 | a) 40 |
| b) Time (seconds) | b) 2 to 4 | b) 1 to 5 | b) 3 |
| Dipping into a solution containing the coating composition | | | |
| a) Temperature (°C) | a) 30 to 55 | a) 25 to 65 | a) 45 |
| b) Time (seconds) | b) 6 to 8 | b) 5 to 10 | b) 7 |

The coating formed on the skin-contacting surface of the elastomeric article has a thickness in the range from 0.1 microns to 1.2 microns. Typically, the coating formed on the skin-contacting surface of the elastomeric article has a thickness of about 0.2 microns.

In accordance with the present invention, an illustrative (coating) solution containing the coating composition includes:
i. 0.5% to 11% by weight/volume of at least one cooling agent;
ii. 0.5% to 11% by weight/volume of at least one non-ionic surfactant; and
iii. water, wherein rest of the solution is made of water.

Preferably, the coating composition comprises about 1% to 6% by weight/volume of cooling agent and about 1% to 6% by weight/volume of non-ionic surfactant. The cooling agent and the non-ionic surfactant are present in a ratio of about 1:1.

The cooling agent is selected from essential peppermint oil, menthol crystals and menthol flakes, wherein source of the essential peppermint oil is from the peppermint plant, preferably the cooling agent is essential peppermint oil.

The non-ionic surfactant is selected from polysorbate 20, octylphenoxypoly (ethyleneoxy) ethanol, branched and liquid lecithin, preferably the non-ionic surfactant is polysorbate 20.

According to the present invention, the gloves, finger cots or any other elastomeric articles coated with the coating composition have a distinct peppermint scent and good tensile strength. TABLE 2, TABLE 3 and TABLE 4 show a comparison of mechanical properties of an elastomeric glove and a finger cot having at least one skin-contacting surface coated with the coating composition prepared in accordance with the present invention with a conventional glove and a conventional finger cot.

**TABLE 2: Mechanical properties of nitrile glove as per ASTM D6319**

| **ASTM D6319** | **Tensile strength (MPa)** | | **Elongation at break (%)** | | **Modulus @ 500% (MPa)** | |
|---|---|---|---|---|---|---|
| | Before aging | After aging | Before aging | After aging | Before aging | After aging |
| Standard requirement (Minimum) | 14 | 14 | 500 | 400 | Nil | Nil |
| Conventional glove | 21.13 | 23.02 | 671.2 | 633.1 | 6.48 | 7.36 |
| Coated glove according to the | 20.51 | 22.49 | 656.3 | 628.9 | 6.31 | 7.88 |
| present invention | | | | | | |

**TABLE 3: Mechanical properties of nitrile glove as per EN 455**

| **EN 455** | **Force at break (N)** | |
|---|---|---|
| | Before aging | After aging |
| Standard requirement | 6 | 6 |
| Conventional glove | 6.07 | 6.52 |
| Coated glove according to present invention | 6.11 | 6.43 |

**TABLE 4: Mechanical properties of finger cot as per ASTM 3772**

| **ASTM 3772** | **Tensile strength (MPa)** | **Elongation at break** (**%)** |
|---|---|---|
| | Before aging | Before aging |
| Standard requirement (Minimum) | 24 | 750 |
| Conventional finger cot | 28.8 | 782 |
| Coated finger cot according to the present invention | 28.1 | 790 |

As seen in TABLES 2, 3 and 4, the coated glove and coated finger cot prepared in accordance with the present invention have passed the minimum requirement of ASTM D6319, EN 455 and ASTM 3772 respectively, required for before and after aging (100°C for 22 hours).

TABLE 5 shows the test results on the sensory evaluation of the cooling sensation of the coated glove from 20 participants. TABLE 6 shows the test results on the sensory evaluation of the cooling sensation of the coated finger cot from 10 participants. The test was conducted in a controlled environment of 22°C ± 1°C; at relative humidity (RH) between 40% to 60%. For each participant, the gloves and finger cots, both coated and control, were provided on left and right hand randomly. Each participant provided a rating based on his/her sensory evaluation after wearing the glove and finger cot based on a subjective rating scale from 0 to 5. An average of the test results and the rating scale are seen in TABLES 5 and 6 herein below for coated glove and coated finger cot respectively.

**TABLE 5: Average of sensory evaluation results for coated glove**

| **Glove samples** | **Cooling sensory effect (scale of 0 to 5)** | | |
|---|---|---|---|
| | **Immediately after wearing** | **After 10 minutes of wearing** | **After 30 minutes of wearing** |
| Control glove | 0.00 | 0.00 | 0.00 |
| Coated glove according to the present invention | 3.25 | 4.05 | 3.45 |
| 0: No cooling effect | | | |
| 1: Very slight cooling effect | | | |
| 2: Slight cooling effect | | | |
| 3: Moderate cooling effect | | | |
| 4: Strong cooling effect | | | |
| 5: Very strong cooling effect | | | |

**TABLE 6: Average of sensory evaluation results for coated finger cot**

| **Glove samples** | **Cooling sensory effect (On the scale of 0 to 5)** | | |
|---|---|---|---|
| | **Immediately after worn** | **After 10 minutes of wearing** | **After 30 minutes of wearing** |
| Control finger cot | 0.0 | 0.0 | 0.0 |
| Coated finger cot according to the present invention | 2.6 | 2.9 | 1.6 |
| 0: No cooling effect | | | |
| 1: Very slight cooling effect | | | |
| 2: Slight cooling effect | | | |
| 3: Moderate cooling effect | | | |
| 4: Strong cooling effect | | | |
| 5: Very strong cooling effect | | | |

Based on the sensory evaluation results, it is seen that the coated glove and coated finger cot produced by the present invention can improve user's sensory experience of wearing the glove or other coated elastomeric articles prepared based on the present invention, thus making the wearing experience comfortable. Also, the coated glove or coated elastomeric article as proposed by the present invention does not vary the hand temperature.

The terminology used herein is for the purpose of describing particular example embodiments only and is not intended to be limiting. As used herein, the singular forms "a", "an" and "the" may be intended to include the plural forms as well, unless the context clearly indicates otherwise. The terms "comprises", "comprising", "including" and "having" are inclusive and therefore specify the presence of stated features, integers, steps, operations, elements and/or components, but do not preclude the presence or addition of one or more other features, integers, steps, operations, elements, components and/or groups thereof.

The methods, steps, processes and operations described herein are not to be construed as necessarily requiring their performance in the particular order discussed or illustrated, unless specifically identified as an order of performance. It is also to be understood that additional or alternative steps may be employed. The use of the expression "at least" or "at least one" suggests the use of one or more elements, as the use may be in one of the embodiments to achieve one or more of the desired objects or results.

## Claims

1. An elastomeric article having at least one skin-contacting surface coated with a water-based coating composition, wherein the coating composition comprises:
i. 0.5% to 11% by weight/volume of at least one cooling agent; and
ii. 0.5% to 11% by weight/volume of at least one non-ionic surfactant.

2. The elastomeric article as claimed in claim 1, wherein the coating (formed by the coating composition) on the skin-contacting surface of the elastomeric article has a thickness in the range from 0.1 microns to 1.2 microns.

3. The elastomeric article as claimed in claim 1 or 2, wherein the coating composition comprises about 1% to 6% by weight/volume of the cooling agent.

4. The elastomeric article as claimed in any of claims 1 to 3, wherein the coating composition comprises about 1% to 6% by weight/volume of the non-ionic surfactant.

5. The elastomeric article as claimed in any of claims 1 to 4, wherein (i) and (ii) are present in a ratio of about 1:1.

6. The elastomeric article as claimed in any of claims 1 to 5, wherein the cooling agent is selected from essential peppermint oil, menthol crystals or menthol flakes.

7. The elastomeric article as claimed in claim 6, wherein the essential peppermint oil is plant based essential peppermint oil.

8. The elastomeric article as claimed in any of claims 1 to 7, wherein the non-ionic surfactant is selected from polysorbate 20, octylphenoxypoly (ethyleneoxy) ethanol, branched or liquid lecithin.

9. The elastomeric article as claimed in any of claims 1 to 8, wherein the coating composition comprises iii. water, wherein the rest of the composition is made of water.

10. A process for making a coated elastomeric article comprising the step of applying a coating composition as defined in any of claims 1 to 9 onto at least one skin-contacting surface of the elastomeric article.

11. The process as claimed in claim 10, further comprising the steps of former cleaning, coagulant dipping, drying, first latex dipping, drying, second latex dipping, drying, pre-leaching, beading, drying, cooling, chlorinating, post-leaching, coating with said coating composition, drying and stripping the elastomeric article from the former, wherein the coating is carried out online.

12. The process as claimed in claim 10 or 11,
wherein the step of coating includes spraying a solution containing the coating composition on a skin-contacting surface of the elastomeric article, wherein the temperature of the solution is maintained in the range between 20°C to 55°C and the spraying is carried out for a period between 1 second to 5 seconds, or wherein the step of coating includes spraying a solution containing the coating composition on a skin-contacting surface of the elastomeric article, wherein the temperature of the solution is maintained in the range between 25°C to 50°C and the spraying is carried out for a period between 2 seconds to 4 seconds, or wherein the step of coating includes spraying a solution containing the coating composition on a skin-contacting surface of the elastomeric article, wherein the temperature of the solution is maintained at 40°C and the spraying is carried out for 3 seconds.

13. The process as claimed in claim 10 or 11,
wherein the step of coating includes dipping a skin-contacting surface of the elastomeric article into a solution containing the coating composition, wherein the temperature of the solution is maintained in the range between 25°C to 65°C and the dipping is carried out for a period between 5 seconds to 10 seconds, or
wherein the step of coating includes dipping a skin-contacting surface of the elastomeric article into a solution containing the coating composition, wherein the temperature of the solution is maintained in the range between 30°C to 55°C and the dipping is carried out for a period between 6 seconds to 8 seconds, or
wherein the step of coating includes dipping a skin-contacting surface of the elastomeric article into a solution containing the coating composition, wherein the temperature of the solution is maintained at 45°C and the dipping is carried out for 7 seconds.
